# EUROPEAN PATENT SPECIFICATION

(11) **EP 0 952 224 B1**
(45) Date of publication and mention of the grant of the patent: **23.06.2004**
(21) Application number: 99302909.9
(22) Date of filing: 14.04.1999
(51) Int. Cl.: C12N 15/82, C12N 15/53, A01H 5/00, A61K 7/00, A61K 7/28, A61K 38/44

(54) **Method for producing transgenic cucumber that produces high levels of superoxide dismutase**
Verfahren zur Herstellung transgener Gurken mit hohem Superoxid-dismutasegehalt
Procédé de préparation de concombre transgénique contenant de hauts niveaux de superoxyde dismutase

(30) Priority: 14.04.1998 KR 9813205; 21.08.1998 KR 9833947; 06.04.1999 KR 9911848
(43) Date of publication of application: 27.10.1999
(73) Proprietor: KOREA INSTITUTE OF SCIENCE AND TECHNOLOGY, Seoul 136-130 (KR)
(72) Inventor: Kim, Jae-Whune, Yusong-ku, Taejon-si 305-345 (KR); Lee, Haeng-Soon, Yusong-ku, Taejon-si 305-333 (KR); Kwon, Suk Yoon, Yusong-ku, Taejon-si 305-333 (KR); Kwak, Sang Soo, Yusong-ku, Taejon-si 305-345 (KR)
(74) Representative: Hall, Marina

(56) References cited:
- EP-A- 0 412 912
- EP-A- 0 670 366
- LIN CHI-TSAI ET AL: "Cloning and characterization of a cDNA encoding the cytosolic copper/zinc-superoxide dismutase from sweet potato tuberous root." PLANT MOLECULAR BIOLOGY, vol. 23, no. 4, 1993, pages 911-913, XP002159220 ISSN: 0167-4412
- OHKAWA JUN ET AL: "Structure of the genomic DNA encoding cucumber ascorbate oxidase and its expression in transgenic plants." PLANT CELL REPORTS, vol. 13, no. 9, 1994, pages 481-488, XP000979465 ISSN: 0721-7714
- ALLEN R D: "DISSECTION OF OXIDATIVE STRESS TOLERANCE USING TRANSGENIC PLANTS" PLANT PHYSIOLOGY,US,AMERICAN SOCIETY OF PLANT PHYSIOLOGISTS, ROCKVILLE, MD, vol. 107, 1995, pages 1049-1054, XP002072721 ISSN: 0032-0889
- DE BLOCK M. ET AL: 'ENGINEERING HERBICIDE RESISTANCE IN PLANTS BY EXPRESSION OF A DETOXIFYING ENZYME' EMBO JOURNAL vol. 6, no. 9, 1987, pages 2513 - 2418
- SEN GUPTA A. ET AL: 'Overexpression of superoxide dismutase protects plants from oxidative stress.' PLANT PHYSIOLOGY vol. 103, 1993, pages 1067 - 1073

## Description

### Field of the Invention

The present invention relates to transgenic cucumber plants which produce high levels of superoxide dismutase (hereinafter, referred to as SOD) and a method for producing said transgenic cucumber plants. More particularly, said transgenic plant of the present invention is produced by co-culturing the hypocotyl section of a seedling with *Agrobacterium* transformant and by regenerating the infected hypocotyl section using a novel method for inducing adventitious shoot, wherein said *Agrobacterium* transformant contains an expression vector for transforming plants. Said expression vector comprises a fruit-dominant promoter of cucumber ascorbate oxidase gene (hereinafter, referred to as ASO promoter), SOD gene isolated from cassava, and a herbicide-resistant bar gene. Since the transgenic cucumber of the present invention is efficiently produced, maintaining higher SOD activity in the fruits, it can be used as materials for cosmetics, as additives in functional foods and medicines as well as exhibiting tolerances to herbicides and environmental stresses.

In response to environmental stresses as well as the biological stresses induced by pathogens, insects, viruses and so on, most living organisms including plants convert oxygen to reactive oxygen species such as superoxide anion radical, hydrogen peroxide, and hydroxyl radical. Superoxide anion radical (·O₂) is generated from the reaction of molecular oxygen with free electron. In the presence of iron, hydrogen peroxide is converted to hydroxyl radical (·OH), the most toxic reactive oxygen species. These reactive oxygen species are so reactive that they cause serious physiological damages to the organisms.

To eliminate reactive oxygen species, living organisms have various antioxidant systems, which include macromolecular antioxidant enzymes such as SODs, peroxidases, catalases and so on, and other antioxidant molecules such as vitamin C, vitamin E, glutathione and so on. SODs are ubiquitous enzymes converting superoxide anion radicals to hydrogen peroxide. To prevent the formation of hydroxyl radicals, either peroxidases or calatases scavenge hydrogen peroxide, converting it to water. Thus, SODs play important roles in the antioxidant system and defense mechanism of living organisms, scavenging superoxide anion radicals and preventing the formation of hydroxyl radicals.

Since SODs are important factors improving tolerance in organisms to environmental stresses, it has been attempted to use SOD as ingredients of medicines, foods, cosmetics, and the like. In addition, transgenic plants into which SOD gene is transferred, have been produced in order to develop plants which are tolerant to various environmental stresses, such as ozone, low temperature, herbicides and so on (*Plant Physiol*., 107, 1049-1054, 1995; USP 5538878).

It has been reported that SOD is effective in the treatment of arthritis, rheumatism, ischemic heart disease, radiation hazard and the like. It has been recently revealed that, when applied to UV-irradiated skin, SOD causes the recovery of the damaged skin (*Experimental Dermatology,* 6, 116-121, 1997). These advances have lead to the development of medicines, which comprise SOD as an effective ingredient, by pharmaceutical companies in America, Japan, and so on. Additionally, SOD-containing cosmetics which prevent aging of the skin have been developed and commercialized in Korea (SOD and active oxygen modulators: pharmacology and clinical trials, NIHON-IGAKUKAN, 1989).

However, since purified SOD loses its activity quickly, there has been a limit to the effective production of medicines or cosmetics in which SOD activity is maintained.

The production of a protein extract rich in Superoxide Dismutase (SOD) obtained from melon (*Cucumis melo*) for medical, cosmetics and/or alimentary uses is disclosed in EP 0670366 A.

To solve such a problem, the inventors of the present invention have developed plants which produce high levels of SOD, and especially have developed the plant bioreactor system by which SOD is overexpressed in edible plant tissue, instead of using isolated SOD as an ingredient of medicines or cosmetics.

The plant bioreactor system overexpressing a specific gene in plant tissue has been occasionally reported, e. g. edible vaccines produced from transgenic bananas into which the vaccine gene is introduced (*Bio*/*Technology,* 13, 379-392, 1995) . However, there has never been an attempt to overexpress SOD gene in edible plants.

Cucumber (*Cucumis sativus* L.) is a high value-added food, and massage packs using cucumber are utilized widely, because of the various properties of cucumber such as the supply of nutrients, the removal of wastes and keratin, suppressive effect on inflammation, whitening, moisturizing, and the like (Techniques for cultivation of cucumber, Kurye Cucumber Experimental Station in Korea, 1997). Although cucumber has such advantages, SOD content (units/mg protein) in cucumber -fruits is much lower than those in other tissues.

Thus, if SOD activity is elevated through the transfer of SOD gene into cucumber fruits, the resulting transgenic cucumber will synergistically acquire a preferable trait of higher SOD activity, in addition to the known benefits of cucumber. With this notice, the inventors of the present invention have developed the plant bioreactor system through the production of transgenic plants overexpressing SOD. Particularly, the SOD gene in the form of an expression vector for transforming plants is introduced into *Agrobacterium,* and plant tissue is co-cultured with the *Agrobacterium* transformant, then regenerated to adult plants which overexpress the SOD gene.

In order to produce the transgenic plants efficiently, plant tissues, into which foreign genes are introduced, should be regenerated. Thus, the inventors of the present invention have established novel and more efficient methods for inducing adventitious shoots from the cultured hypocotyl section, repeatedly and regardless of the species or cultivar of the seed plants. The present invention is performed by the production of said SOD transgenic plants, which is regenerated through a novel method for inducing adventitious shoot.

### Brief Description of the Drawings

**Figure 1** depicts a vector system of the present invention,
**Figure 2** depicts the processes in which the regenerated plants are prepared, where
Figure 2a represents the induction of adventitious shoots from hypocotyl section,
Figure 2b represents the development of leaves from said adventitious shoots,
Figure 2c represents the induction of roots, and
Figure 2d represents the regenerated plants in pots,
**Figure 3** depicts the relationship between the ages of cucumber seedlings and the frequencies of adventitious shoot induction from various cultured tissues, where
   2C + H represents the induction frequency from the hypocotyl section of seedlings with two intact cotyledons,
   1C + H represents the induction frequency from the hypocotyl section of seedlings with one intact cotyledon,
   (1/2C + 1/2C) + H represents the induction frequency from the hypocotyl section of seedlings with two half-cotyledons, and
   1/2C represents the induction frequency from cotyledon segments,

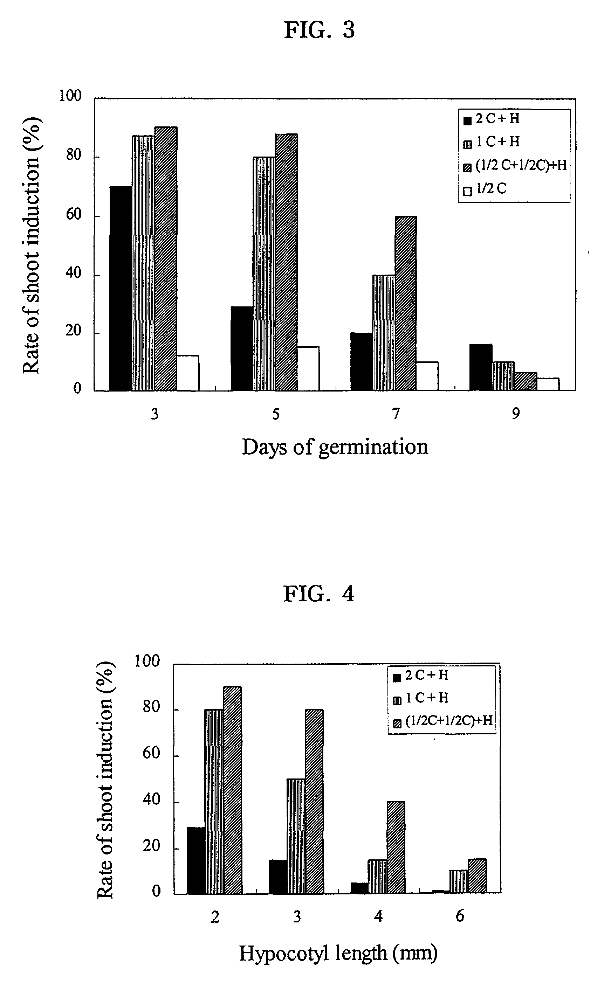

**Figure 4** depicts the relationship between the hypocotyl length of 5 DAG (day-after-germination) seedlings and the frequency of adventitious shoot induction from the hypocotyl section, where
   2C + H represents the induction frequency from the hypocotyl section of seedlings with two intact cotyledons,
   1C + H represents the induction frequency from the hypocotyl section of seedlings with one intact cotyledon, and
   (1/2C + 1/2C) + H represents the induction frequency from the hypocotyl section of seedlings with two half-cotyledons,

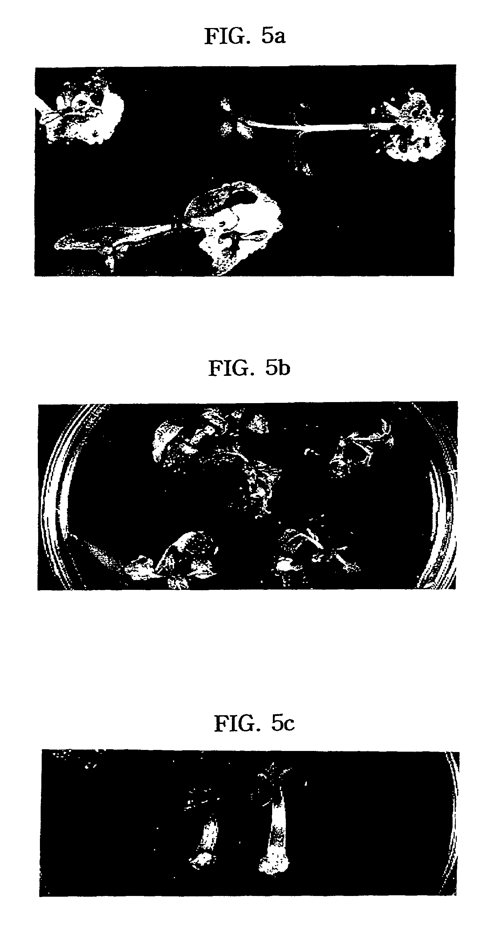

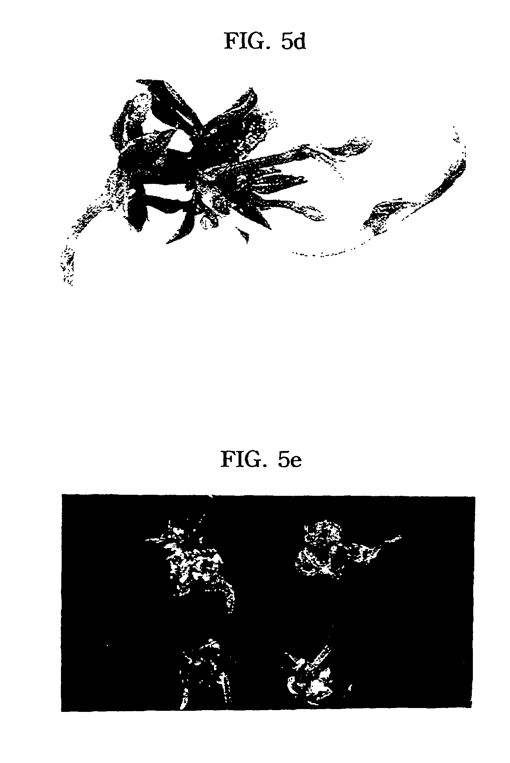

**Figure 5** depicts the adventitious shoots induced efficiently from the hypocotyl section of various seedlings such as Chinese matrimony vine, red pepper, melon, bean, and spring onion, where
Figure 5a represents the adventitious shoots induced efficiently from the hypocotyl section of Chinese matrimony vine seedlings,
Figure 5b represents the adventitious shoots induced efficiently from the hypocotyl section of red pepper seedlings,
Figure 5c represents the adventitious shoots induced efficiently from the hypocotyl section of melon seedlings,
Figure 5d represents the adventitious shoots induced efficiently from the hypocotyl section of bean seedlings, and
Figure 5e represents the adventitious shoots induced efficiently from the hypocotyl section of spring onion seedlings,

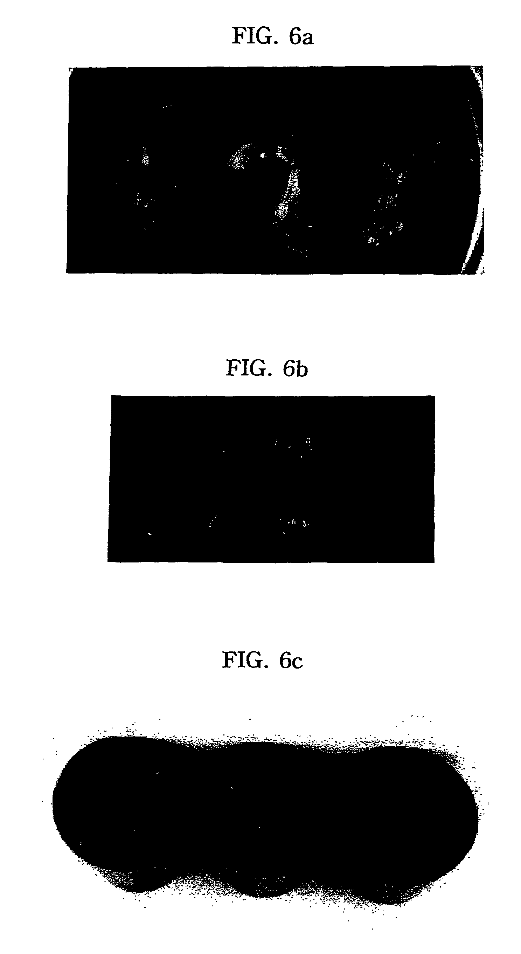

**Figure 6** depicts the processes in which SOD transgenic cucumbers are prepared through the adventitious shoots induced by co-culturing the hypocotyl section with *Agrobacterium* transformant, where
Figure 6a represents transformed adventitious shoots on hypocotyl section,
Figure 6b represents the rooted adventitious shoots, which is severed from the seedlings, and
Figure 6c represents the regenerated transgenic cucumbers in pots,

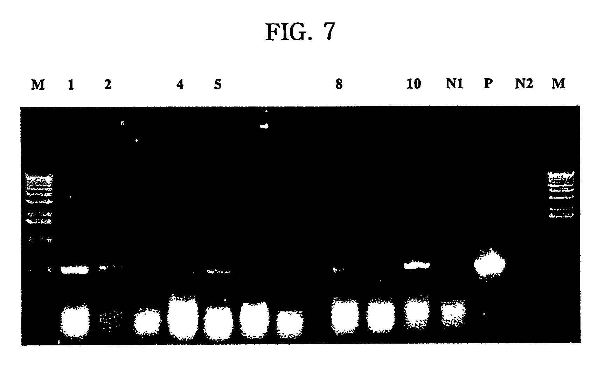

**Figure 7** depicts the introduction of SOD gene into the genomes of said transgenic cucumbers, by polymerase chain reaction (hereinafter, referred to as PCR) and subsequent DNA gel electrophoresis, where
   Lanes 1, 2, 4, 5, 8, 10 represent PCR products from transformants,
   N1 represents PCR products from non-transformants,
   N2 represents negative control of PCR, in which no template is added,
   M represents DNA size marker, and
   P represents positive control of PCR, in which vector ASOp+mSOD1/pGPTV-Bar is used as template.

### Detailed Description of the Preferred Embodiments

The present invention provides a transgenic cucumber plant that produces high levels of SOD.

The present invention provides a method for producing said transgenic cucumber plant, wherein SOD gene in the form of an expression vector for transforming plants is introduced into *Agrobacterium,* and then plant tissue is co-cultured with the resulting *Agrobacterium* transformant.

The expression vector for transforming plants in the present invention is ASOp+mSOD1/pGPTV-Bar, which comprises CuZn SOD cDNA (mSOD1) isolated from the cultured cells of cassava, a herbicide(Basta)-resistant bar gene as a selection marker, and ASO promoter.

The *Agrobacterium* transformant includes the expression vector ASOp+mSOD1/pGPTV-Bar.

The *Agrobacterium* transformant in the present invention is preferably *Agrobacterium tumefaciens* LBA 4404 (ASOp+mSOD1/pGPTV-Bar).

The present invention provides a method for producing regenerated plants, comprising the following steps :
a) the severing of the upper hypocotyls of germinated seedlings,
b) the induction of adventitious shoots from the hypocotyl section,
c) the induction of roots from the induced adventitious shoots, and
d) the acclimatization of resulting plantlets to the soil.

The present invention provides a method for producing regenerated transgenic plants, wherein a useful gene is transferred into the hypocotyl section, and then adventitious shoot is induced from there.

In addition, the present invention provides uses of said SOD transgenic plants as materials for cosmetics including massage packs, as additives in functional foods, and for Basta-resistant plant.

Hereinafter, the present invention is described in detail.

To produce said transgenic plant which produces SOD dominantly in cucumber, the present invention preferably uses an expression vector for transforming plants, which comprises ASO promoter, mSOD1 gene, and bar gene. Said vector is introduced into *Agrobacterium* to produce *Agrobacterium* transformant, with which plant tissue is co-cultured. In said co-culture process, organ culture is exploited to induce adventitious shoot growth from the hypocotyl section of germinated seedlings and to induce root growth from the adventitious shoot, leading to regeneration of plants.

Said regeneration method using organ culture, comprises :
a) the germination of sterilized seeds on MS medium without plant growth regulators,
b) the severing of the hypocotyl of germinated seedlings,
c) the induction of adventitious shoots from the hypocotyl section,
d) the induction of roots from the adventitious shoots, and
e) the acclimatization of the plantlets to soil.

In said process of adventitious shoot induction, the hypocotyl section is cultured under lights, which occasionally leads to poor induction from the hypocotyl section of some plant species. In this case, induction frequency can be elevated, by culturing the plants under darkness for specified period and then transferring them to lights.

In addition, the preferable age of seedlings is 3-5 DAG (days after germination), the preferable hypocotyl length in the severing process is 2-3 mm, and the preferable seedlings have either one intact cotyledon or two half-excised cotyledons, although these preferences are variable according to plant species.

As shown above, the present invention provides a method for producing normal, regenerated plants by inducing adventitious shoots from the hypocotyl section. By said method, adventitious shoots are abundantly obtained from the hypocotyl section of cucumber (*Cucumis sativus* L.) plants.

Therefore, said method for inducing adventitious shoots is effectively applicable to plant regeneration, which is the essential process for the propagation of useful plants and the development of transgenic plants.

The novel method for inducing adventitious shoots provides relative advantages to those methods already established (ie where only cotyledon segments are cultured), as shown by the following :
a) In the method using cotyledon segments, adventitious shoots may originate from the leaf primordia which are accidentally attached to the cotyledon segments. Employing hypocotyl sections as the cultured material precludes this possibility.
b) If cotyledon segments are cultured from the seedlings of the Cucurbitaceae family such as cucumber, the curled leaves with multiple layers are often induced, and the cultured explants do not develop the shoot apical meristem, or, if any develops, the explant often ceases to grow and shows collective dwarfism (*Plant Cell Report*, 9, 559-562, 1991; *J. Amer. Soc. Hort. Sci*., 118, 151-157, 1993). On the contrary, the adventitious shoots induced from hypocotyl sections in the present invention scarcely show any signs of either undifferentiation or collective dwarfism.

In addition, said method for inducing adventitious shoots is applicable to the efficient regeneration of transgenic plants (herein, cucumber plants), by transferring useful foreign genes into hypocotyl sections and then inducing adventitious shoots from there. More particularly, the gene transfer is mediated by the infection of the hypocotyl section with *Agrobacterium tumefaciens* strain, which is transformed with the useful foreign gene.

In the present invention, said method for inducing adventitious shoots is used to produce cucumber plants overexpressing SOD gene. In detail, in order to produce said SOD transgenic plants, an expression vector for transforming plants with SOD gene is introduced into *Agrobacterium,* and the expression vector is transferred into plant tissue by co-culturing the hypocotyl section with said *Agrobacterium* transformant.

The method for producing said transgenic plant, which is regenerated by the method for inducing the adventitious shoot, comprises :
a) constructing an expression vector, which comprises superoxide dismutase gene under the control of ascorbate oxidase promoter and the Basta resistant gene as a selection marker,
b) producing *Agrobacterium* transformants by transferring said vector into *Agrobacterium*,
c) co-culturing said *Agrobacterium* transformants with plant tissue for the enhancement of adventitious shoot induction wherein said co-culturing process comprises :
   (i) severing the hypocotyl of germinated seedlings to eliminate the lower part of the seedlings,
   (ii) infecting the hypocotyl section of the severed seedlings with *Agrobacterium* transformants,
   (iii) culturing the severed seedlings on à medium for adventitious shoot induction, with the hypocotyl section contacting the medium, wherein the medium is MS medium supplemented with zeatin, and
d) regenerating the transformed tissue into adult transgenic plants.

*Agrobacterium* used in b) process include all the species of *Agrobacterium*, into which the expression vector for transforming plants is introduced.

In order to produce cucumber containing SOD abundantly and to use it as material for massage packs etc., the present invention exploits expression vectors for transforming plants. This expression vector comprises a promoter (ASO promoter) expressed dominantly in cucumber fruits (*Proc. Natl. Acad. Sci. USA,* 86, 1239-1243, 1989; *Ann. N. Y. Acad. Sci*., 721, 245-247, 1994), an SOD gene (mSOD1) isolated from cassava cultured cells, and a herbicide-resistant gene (bar).

Although SOD genes have been isolated from more than 30 plant species, a CuZn SOD gene (mSOD1, see SEQ ID NO. 1) which is isolated first from cassava (*Manihot esculenta*) cultured cells, is an example of a SOD gene used to construct said expression vector of the present invention.

Basta-resistant gene, bar, is used as a selection marker for said expression vector. Non-selective herbicide Basta is broadly used, since it has high herbicidal activity and causes less soil pollution than other herbicides do. Phosphinothricin, a composite of Basta, is synthesized by *Streptomyces hygroscopicus,* and inhibits strongly the glutamine biosynthesis of plants. Application of phosphinothricin or Basta to plants, elevates ammonia level in the plants, leading to the browning and withering of the plants. bar encodes phosphinothricin acetyltransferase, an enzyme detoxifying phosphinothricin (*EMBO*, 6, 2519-2523, 1987). Therefore, bar has been frequently used to select transformed plants as well as to develop herbicide-resistant plants.

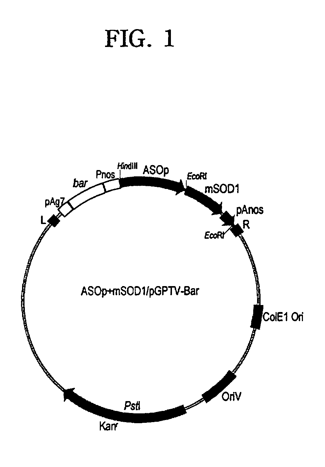

The expression vector for transforming plants in the present invention is ASOp+mSOD1/pGPTV-Bar (see figure 1), which comprises mSOD1, bar, ASO promoter, and the like. This vector can be introduced into *Agrobacterium*, preferably *Agrobacterium tumefaciens*.

In the present invention, the strain *Agrobacterium tumefaciens* LBA 4404 is used as an *Agrobacterium* strain, into which said expression vector is introduced. The *Agrobacterium* transformant in the present invention was designated as *Agrobacterium tumefaciens* LBA 4404 (ASOp+mSOD1/pGPTV-Bar), and deposited in Korean Collection for Type Cultures (KCTC) on March 6, 1999 (accession NO. KCTC 0585BP).

The cucumbers of SOD transgenic plants, which are produced through the method of the present invention, show higher antioxidant activity, thus can be used as materials for cosmetics, additives in functional foods, or medicines, particularly as materials of cosmetics, for example, massage packs.

Additionally, since the SOD transgenic cucumber plants in the present invention show tolerance to herbicide (Basta) as well as the higher antioxidant activity, they can be efficiently cultivated in stressful environments.

Practical and presently preferred embodiments of the present invention are illustrated in the following Examples.

### Examples

### <Example 1> Adventitious shoot induction and plant regeneration from hypocotyl section of cucumber seedlings

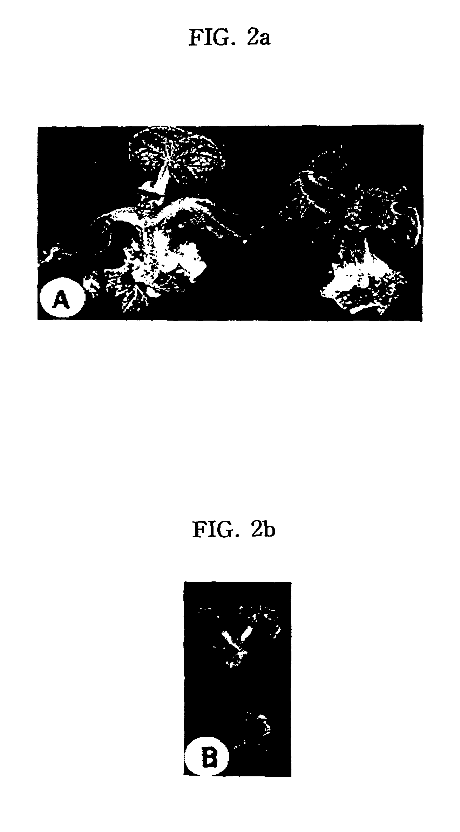

In order to regenerate plants through the organogenesis, the seeds of five cucumber cultivars (Yoroomsamchuck, Eunsongbakdadagi, Bakbongdadagi, Chosengnakhap, Changhyongnakhap) were soaked in distilled water for 1 hour, sterilized in 75% ethanol for 1 minute and in 2% sodium hypochlorite for 15 minutes, and washed with sterile water three times. The sterilized seeds were germinated on MS media without growth regulators (Murashige and Skoog, *Physiol. Plant.* 15, 473-497, 1962) at 25 ± 1 °C, with a photoperiod of 16 hours under fluorescent light (intensity : about 15 µmol m⁻²·s⁻¹) and 8 hours under darkness. After various periods of germination, the upper region of the hypocotyls was severed to discard the lower part of seedlings. The upper part of the severed seedlings was transferred to medium for adventitious shoot induction (MS medium supplemented with 2.0 mg/L zeatin), with the hypocotyl section of the seedlings contacting the medium. Seedlings on the medium were cultured, with a photoperiod of 16 hours under fluorescent light (intensity : about 15 µmol m⁻²·s⁻¹) and 8 hours under darkness (see figure 2a) . After 3 weeks, the adventitious shoots were induced from the hypocotyl sections of the seedlings, and the frequency of adventitious shoot induction was investigated (see below).

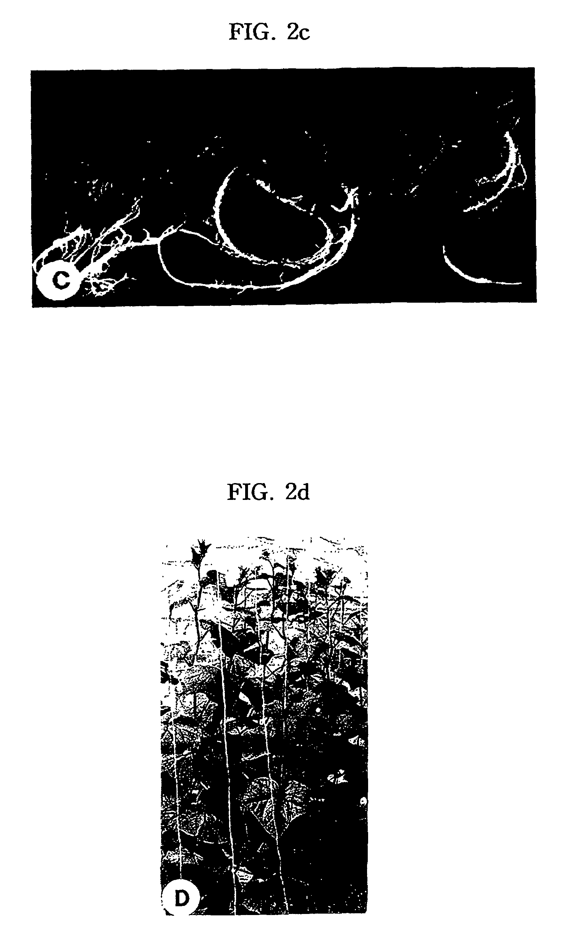

The adventitious shoots developing 1 or 2 leaves (see figure 2b) were transferred to media for root induction, the MS media supplemented with 1.0 mg/L IAA (indole-3-acetic acid), and cultured for 2 weeks to be rooted easily (see figure 2c). Rooted plantlets with 2 or 3 leaves were transferred into pots, and acclimatized to soil under the condition of about 80% humidity. Regenerated plants could be grown to normal, fertile adult plants (see figure 2d).

To assess the frequencies of adventitious shoot induction from the hypocotyl section according to age (i.e. Days After Germination : hereinafter, referred to as DAG ) of cucumber seedlings used, the upper hypocotyls of 3, 5, 7 or 9 DAG seedlings were severed to discard the lower part of the seedlings, leaving 2 mm-length hypocotyls. The severed seedlings were cultured on the media for adventitious shoot induction as described earlier. As a result, hypocotyl section of 3 DAG seedlings efficiently produced adventitious shoots, whether the severed seedling had one or two cotyledons, or whether it had intact cotyledons or half-cotyledons (see figure 3). In the case of seedlings with two cotyledons, 3 DAG seedlings produced adventitious shoots with high frequency (about 70%), while 5 DAG seedlings did not (below 30%) (see figure 3). Thus, the most preferable materials to culture are the 3-5 DAG seedlings which have either one cotyledon or two half-cotyledons, if one intends to induce adventitious shoots efficiently from hypocotyl sections of cucumber seedlings.

To investigate the relationship between the length of hypocotyls and the efficiency of adventitious shoot induction from the hypocotyl section, the hypocotyls of 5 DAG seedlings were excised to 2, 3, 4, or 6 mm-length, and cultured on MS media supplemented with 2.0 mg/L zeatin. As a result, the longer the hypocotyls, the smaller the amount of adventitious shoots induced from them (see figure 4), suggesting that cells in the upper hypocotyl are competent for adventitious shoot formation. In the case of seedlings with two intact cotyledons, the efficiency of induction was remarkably reduced according to the increased length of the hypocotyls. In addition, seedlings with 6 mm hypocotyls rooted on the media, which is a reasonable result because the presence of hypocotyl and two cotyledons may attenuate the effect of exogenous zeatin, and because of the plant hormone auxin synthesized in the shoot apical meristem, may induce root formation.

Again, the size of cotyledons has an effect on adventitious shoot induction : the sections of 2 mm hypocotyls produced adventitious shoots with similar efficiency whether the severed seedlings had one or two cotyledons or whether it had intact cotyledons or half-cotyledons, while the sections of 3 mm hypocotyl with two half-cotyledons showed still higher efficiency than with two intact cotyledons (see figure 4). The results shown in figure 3 and figure 4 suggest that the number and the size of cotyledons have significant effects on adventitious shoot induction from the hypocotyl section.

### <Comparative Example 1> Adventitious shoot induction from cucumber cotyledon segment

In order to regenerate plants through the organogenesis, the seeds of five cucumber cultivars (Yoroomsamchuck, Eunsongbakdadagi, Bakbongdadagi, Chosengnakhap, Changhyongnakhap) were soaked in distilled water for 1 hour, sterilized in 75% ethanol for 1 minute and in 2% sodium hypochlorite for 15 minutes, and washed with sterilized water three times. The sterilized seeds were germinated on MS media without growth regulators at 25 ± 1 °C, with a photoperiod of 16 hours under fluorescent light (intensity : about 15 µmol m⁻²·s⁻¹) and 8 hours under darkness. The basal region (5 mm × 5 mm) of the cotyledons of 5 or 7 DAG seedlings was cut and transferred onto medium for culture of cotyledon segments, which is an MS medium supplemented with 3% sucrose, 0.4% Phytagel, and cytokinin [1.0 or 2.0 mg/L zeatin, 1.0 mg/L 6-benzyladenine (BA), and 1.0 or 3.0 mg/L kinetin] with/without 0.1 or 0.2 mg/L indole-3-acetic acid (IAA). The medium was adjusted to pH 8.5, sterilized by autoclaving for 15 minutes at 121 °C, 1.2 atm, on liquid cycle, and dispensed into 25mL aliquots in Petri dishes (87 mm × 15 mm). The Petri dishes containing five cotyledon segments were cultured at 25 ± 1 °C, with a photoperiod of 16 hours under fluorescent light (intensity : about 15 µmol m⁻²·s⁻¹) and 8 hours under darkness. The cotyledon segments were grown for 4 weeks, and the investigation for the adventitious shoot formation was carried out.

**Table 1.**

| The adventitious shoot formation in the cucumber cotyledon segments which were cultured for 4 weeks in various compositions of plant hormones | | | | | | |
|---|---|---|---|---|---|---|
| Contents in MS media (mg/L) | | | | No. of cotyledon segments used | No. of adventitious shoots induced | The frequency of adventitious shoot induction (%) |
| BA | Kine tin | Zea tin | IAA | | | |
| 1.0 | | | | 315 | 4 | 1.3 |
| 1.0 | | | 0.1 | 720 | 32 | 4.4 |
| | 1.0 | | | 210 | 0 | 0 |
| | 1.0 | | 0.1 | 345 | 7 | 2.0 |
| | | 1.0 | | 324 | 11 | 3.4 |
| | | 1.0 | 0.1 | 379 | 46 | 12.1 1 |
| | | 2.0 | 0.2 | 251 | 27 | 10.7 |
| | 3.0 | | 0.2 | 227 | 13 | 5.7 |

As shown in Table 1, with the method already established in which only cotyledon segments are cultured using the same process described in Example 1, shows at most 20% of induction efficiency (see also figure 3).

### <Comparative Example 2> Plant regeneration from cucumber hypocotyl segments by somatic embryogenesis

In this Example, cucumber seeds were germinated by the same process as described in Comparative Example 1, except for being germinated in a dark room. The 5-10 mm-length hypocotyls of 5 DAG seedlings were severed and cultured on callus induction media [MS media (pH 5.8) supplemented with 3, 5, or 10% sucrose, 0.4% Phytagel, and 1.0 mg/L 2,4-dichlorophenoxy acetic acid (2,4-D)] . Embryogenic calli, which were selected through subcultures on the same media, were maintained and proliferated. In order to induce somatic embryo, the embryogenic callus cultured for 2 weeks on the above media was transferred onto the media without 2,4-D, and further cultured. After 4 weeks, white callus was formed from most of the hypocotyl segments, occasionally pale yellow callus was formed. After the pale yellow callus was separated and subcultured, viscous and clear callus was produced on a contact surface to medium, finally forming yellowish embryogenic callus. However, the induction of this embryogenic callus showed poor efficiency, with a callus induction frequency of about 2.5%.

Only 2.3% of hypocotyls formed embryogenic callus in cucumber cultivars Nakhap series (Chosengnakhap and Changhyongnakhap), when the explants were cultured in darkness and under 3% sucrose condition. In other cultivars, no embryogenic callus was induced, even though sucrose was added to the media up to 10% concentration.

### <Comparative Example 3> Adventitious shoot induction from hypocotyl section of seedlings such as Chinese matrimony vine, red pepper, melon, bean, and spring onion

In this Comparative Example, regenerated plants were produced from adventitious shoots of various seed plants, by a similar process as described in Example 1. These seed plants include Chinese matrimony vine (*Lycium chinense* Mill) as a member of woody plants, red pepper (*Capsicum annuum* L.) as a vegetable, melon (*Cucumis melo* L.) as a member of the Cucurbitaceae family, bean (*Phaseolus vulgaris* L.) as a legume, and spring onion (*Allium fistulosum* L.) as a monocotyledon. The seeds of the above species were sterilized and germinated as described in Example 1. Each cotyledon was removed by halves or completely from germinated seedlings, and their hypocotyls were severed to 2 mm-lengths. Severed seedlings were transferred onto the media for adventitious shoot induction, with the hypocotyl section of the seedlings contacting the media.

The severed hypocotyls of Chinese matrimony vine, red pepper, melon, bean and spring onion abundantly produced adventitious shoots in 3 weeks after transfer onto the media (see figure 5a - 5e), similar to cucumber. While the adventitious shoots in red pepper, bean and spring onion were successfully induced under the same light conditions as in cucumber, the shoots of Chinese matrimony vine and melon were not. Instead of being cultured under light, severed seedlings of Chinese matrimony vine and melon were cultured for the first 2 weeks in darkness, and then transferred to light conditions, so that the adventitious shoots could be frequently induced from these seedlings.

### <Example 2> Cloning and sequencing cassava SOD gene and constructing expression vector for plant transformation

The method for inducing adventitious shoot from hypocotyl sections, illustrated by the above Examples, was exploited to produce transgenic cucumber overexpressing SOD. To do this, an expression vector for transforming plants was constructed (see figure 1). The expression vector in this Example, employs ascorbate oxidase (ASO) promoter as a promoter, mSOD1 isolated from cassava cultured cells as an SOD structural gene, and the Basta-resistant (bar) gene as a selection marker.

At first, in order to construct the above vector, an SOD gene was isolated from cassava (*Manihot esculenta*) and sequenced. SOD genes have been isolated from more than 30 species, but we identified a CuZn SOD gene (mSOD1, described in SEQ ID NO. 1) which is the first SOD gene isolated from cassava cultured cells. To isolate a full-length SOD cDNA, we screened a cDNA library which was constructed in Uni-ZAP vector (Stratagene). Particularly, various pools of cassava cultured cells were screened to select those which expressed high level of SOD. The selected cells were subcultured for 20 days, and mRNA was extracted from the cells. After cDNA was synthesized using the isolated mRNA as template, 4 X 10⁵ pfu (plaque forming unit) of cDNA library was constructed with Gigapack III packaging extract (Stratagene) and the synthesized cDNA, and finally amplified to 2 X 10¹⁰ pfu/mL. To synthesize probes for screening the cDNA library of cassava cultured cells, two degenerate primers, SODF1 (described in SEQ ID NO. 3) and SODR2 (described in SEQ ID NO. 4), were used, whose nucleotide sequences were deduced from the conserved amino acids sequences among various plant SOD genes. The 312 bp product of PCR, in which SODF1 and SODR2, and the cassava cDNA library were used as primers and template respectively, were inserted into EcoRV site of pBluescript vector (Stratagene), and the resulting vector was introduced into *E. coli*. The plasmid was isolated from the *E. coli*, then cleaved at *Xba*I and *Sal*I sites. After selection of plasmids releasing 0.5 kb inserts, the selected plasmids were sequenced to prove them to be a partial SOD gene, and thus could be used to screen the cDNA library of cassava cultured cells. The ³²P-dCTP-labeled probe for screening the cDNA library was synthesized from the 0.5 kb PCR product and used to screen 4 X 10⁶ pfu of the cDNA library twice. As a result, about 50 single plaques were obtained, and 10 selected plaques were sequenced to isolate a full-length CuZn SOD cDNA (mSOD1), which was described in SEQ ID NO. 1.

Isolated mSOD1 was 801 bp cDNA, and had an open reading frame (ORF) comprising 152 amino acid residues. mSOD1 was identified to have a poly-(A) tail and a putative polyadenylation signal, AATAAA sequence, which lay in 170 bp upstream from the poly-(A) tail (see SEQ ID NO. 1). When the deduced amino acid sequence from the above ORF (described in SEQ ID NO. 1 and NO. 2) was compared with other plant CuZn SOD sequences, there were 5 conserved amino acid sequences (MVKAEAVL, PGLHGFHVH, GDTTNGC, DDLGRGGHELS, TGNAGGR), and putative copper-binding sites (His-45, His-47, His-62, His-70, His-79, Asp-82 and His-119).

ASO promoter was inserted to pBluescript vector (Stratagene) at first, in order to construct the expression vector for transforming plants which comprised the isolated mSOD1, the fruit-dominant ASO promoter of cucumber, and Basta-resistant (bar) gene. Particularly, the sequence of the ASO promoter (distributed by Nara Institute of Science and Technology in Japan; *Ann. N. Y. Acad. Sci.* 721, 245-247) was amplified by PCR in which two PCR primers (described in SEQ ID NO. 5 and 6, respectively) were tagged with *Hind*III or *Eco*RI restriction sequences, respectively, at their 5' ends. This PCR product was cleaved by *Hind*III and *Eco*RI restriction enzymes, and inserted into *Hind*III/*Eco*RI site of pBluescript KS vector, to produce vector ASOp/pBluescript.

To insert the isolated mSOD1 into ASOp/pBluescript, the *Pst*I- or *Bam*HI-tagged primers (described in SEQ ID NO. 7 and 8, respectively) and template of the full-length mSOD1 cDNA were used to obtain PCR product for mSOD1, which was then inserted into *Pst*I/*Bam*HI site of the vector ASOp/pBluescript. The resulting vector was designated as ASOp+mSODl/pBluescript.

To insert ASOp+mSOD1 fragment of ASOp+mSOD1/pBluescript into pBI101 (Clontech), a binary vector for transforming plants, the vector ASOp+mSOD1/pBluescript was digested with *Hind*III and *Bam*HI enzymes, and pBI101 with *Hin*dIII and *Sac*I enzymes. After *Bam*HI site and *Sac*I site were made to be blunt ends, the ASOp+mSOD1 fragment was inserted into *Hind*III/*Sac*I site of pBI101. The resulting vector was designated as ASOp+mSOD1/pBI101.

Finally, vector pGPTV-Bar (Plant Mol. Biol. 20, 1195-1197, 1992; Accession NO. ATCC 77391) was employed so that bar gene in pGPTV-Bar might replace the selection marker in pBI101. Particularly, the vector ASOp+mSOD1/pBI101 was digested with *Hin*dIII enzyme and partially digested with *Eco*RI enzyme, producing about 2 kb fragment. This fragment was inserted into *Hin*dIII/*Eco*RI site of pGPTV-Bar, constructing vector ASOp+mSODl/pGPTV-Bar (see figure 1).

To introduce the vector ASOp+mSOD1/pGPTV-Bar into *Agrobacterium tumefaciens* LBA 4404 strain, the vector was mixed with *Agrobacterium tumefaciens* LBA 4404. The mixture was frozen in liquid nitrogen for 5 minutes, and thawed at 37 °C for 5 minutes. After 1 mL of YEP solution (1% bacto-peptone, 1% bacto-yeast extract, 0.5% sodium chloride) was added to the mixture, *Agrobacterium* in the mixture was cultured at 28 °C with shaking and then spread on YEP medium (YEP solidified by 1.5% bacto-agar) containing 50 mg/L kanamycin and 100 mg/L rifampicin. The medium was cultured at 28 °C for 2 days to isolate the colonies of *Agrobacterium* transformants. The transformed strain was isolated, designated as *Agrobacterium tumefaciens* LBA 4404 (ASOp+mSOD1/pGPTV-Bar), and deposited in Korean Collection for Type Cultures (KCTC) on March 6, 1999 (accession NO. KCTC 0585BP).

### <Example 3> SOD gene transfer into cucumber hypocotyl section and the production of transgenic cucumber

The method of the present invention for inducing adventitious shoots is applicable to the efficient production of plants into which the useful gene is introduced. In this Example, hypocotyl sections of cucumber seedlings were infected with the strain *Agrobacterium tumefaciens* LBA 4404 (ASOp+mSOD1/pGPTV-Bar), which mediated the transfer of vector ASOp+mSOD1/pGPTV-Bar into cucumber. At first, cucumber seeds were germinated on MS media in a sterile condition, as described in Example 1. After the upper hypocotyls of the germinated seedlings were severed to discard the lower parts, the hypocotyl sections were infected with *Agrobacterium tumefaciens* LBA 4404 (ASOp+mSOD1/pGPTV-Bar) which had been cultured for 2 days in YEP solution containing 50 mg/L kanamycin and 100 mg/L rifampicin. After draining off the soaked hypocotyl region, the seedlings were transferred to the media for adventitious shoot induction (the MS media supplemented with 2.0 mg/L zeatin), with the hypocotyl section contacting the media. The seedlings on the media were cultured for 4 days, with a photoperiod of 16 hours under fluorescent light (intensity : about 15 µmol m⁻²·s⁻¹) and 8 hours under darkness, so as to infect the hypocotyl section with the *Agrobacterium*. To remove *Agrobacterium* and to select transformed adventitious shoots, the seedlings were transferred onto selective medium (an MS medium supplemented with 2.0 mg/L zeatin, 300 mg/L claforan and 2.0 mg/L Basta). By subculturing the seedlings every week, Basta-resistant adventitious shoots were induced from the hypocotyl sections of the seedlings in 4 weeks after transfer (see figure 6a). After leaves developed from the adventitious shoots, the selected shoots were transferred to media for root induction, the MS media supplemented with 1.0 mg/L IAA (see figure 6b). The resulting transformants were transferred and acclimatized to pots in greenhouse (see figure 6c).

To inquire whether or not the bar gene is stably introduced to the genome of Basta-resistant plantlets, PCR was performed, in which two oligonucleotides (described in SEQ ID NO. 9 and 10) designed from bar gene were used as primers. 10 plantlets with 3-4 leaves were randomly selected, and then their leaves were ground to obtain genomic DNA, which was employed as a template in the PCR. As a result, it was revealed that PCR products from about 60% of Basta-resistant plantlets contained 0.5 kb DNA of bar gene (see figure 7).

In this Example, it was confirmed that the SOD transgenic cucumber could be well developed, by using the novel method of inducing adventitious shoots and using fruit-dominant promoters in cucumber.

### Industrial Applicability

As described above, transgenic cucumber showing far higher SOD activity than existing cultivars, is produced by the present invention through the method described, in which an expression vector for plant transformation, comprising, a cucumber fruit-dominant promoter (ASO promoter), an SOD gene (mSOD1) and a herbicide-resistant gene (bar), is introduced into *Agrobacterium,* the resulting *Agrobacterium* transformant mediates the SOD gene transfer into hypocotyl sections, and tranformed adventitious shoots are induced from the hypocotyl sections to be regenerated. Such SOD transgenic cucumber not only can be used as materials for cosmetics including massage packs, additives in functional foods, medicines and so on, but also shows tolerance to herbicides and various environmental stresses.

In addition, the method for inducing adventitious shoots of the present invention is applicable to propagating cucumber plants or regenerating transformed tissues to adult cucumber plants more faithfully and less laboriously than the established methods. Therefore, the tissues of the cucumber plants recalcitrant against adventitious shoot induction or the induction of somatic embryos can be regenerated and propagated repeatedly through the method of the present invention, which can be used for the propagation of cucumber plants and the development of transgenic cucumber plants.

## Claims

1. A method for producing transgenic cucumber plants comprising the steps of :
a) constructing an expression vector, which comprises superoxide dismutase gene under the control of ascorbate oxidase promoter; and Basta resistant gene as a selection marker,
b) producing *Agrobacterium* transformants by transferring said vector into *Agrobacterium*,
c) co-culturing said *Agrobacterium* transformants with plant tissue for the enhancement of adventitious shoot induction wherein said co-culturing process comprises :
(i) severing the hypocotyl of germinated seedlings to eliminate the lower part of the seedlings,
(ii) infecting the hypocotyl section of the severed seedlings with Agrobacterium transformants,
(iii) culturing the severed seedlings on a medium for adventitious shoot induction, with the hypocotyl section contacting the medium, wherein the medium is MS medium supplemented with zeatin, and
d) regenerating the transformed tissue into adult transgenic plants.

2. The method for producing transgenic cucumber plants according to claim 1, wherein said superoxide dismutase gene is the nucleic acid sequence of SEQ ID NO. 1, which is isolated from cassava.

3. The method for producing transgenic cucumber plants according to claim 1, wherein said expression vector is ASOp+mSOD1/pGPTV-Bar which comprises superoxide dismutase gene under the control of ascorbate oxidase promoter and the Basta-resistant gene as a selection marker.

4. The method for producing transgenic cucumber plants according to any preceding claim, wherein said *Agrobacterium* transformant is *Agrobacterium tumefaciens* LBA 4404 assigned accession NO. KCTC 0585BP, by the Korean Research Institute of Bioscience and Biotechnology.

5. The method for producing transgenic cucumber plants according to any preceding claim, wherein said regenerating process comprises the steps of :
a) inducing adventitious shoots from the infected hypocotyl section on selective medium wherein the selective medium is MS medium supplemented with 2.0 mg/L zeatin, 300 mg/L claforan and 2.0 mg/L Basta,
b) rooting the adventitious shoots induced in a) on a medium for root induction wherein the medium for root induction is MS medium supplemented with 1 mg/L IAA, and
c) acclimatizing the rooted plantlet to soil.

6. The method for producing transgenic cucumber plants according to any preceding claim, wherein said upper hypocotyl is severed to leave 2-3 mm length.

7. The method for producing transgenic cucumber plants according to any preceding claim, wherein said germinated seedlings are severed at 3-5 days after germination, and have one intact cotyledon or two half-excised cotyledons.

8. A transgenic cucumber plant which is transformed with an expression vector comprising superoxide dismutase gene under the control of ascorbate oxidase promoter and the Basta-resistant gene as a selection marker.

9. A use of the transgenic plant according to claim 8 as a material for cosmetics including massage packs, or as an additive in functional foods.

## Patentansprüche

1. Verfahren zur Herstellung transgener Gurkenpflanzen, umfassend die Schritte von:
a) Aufbau eines Expressionsvektors, der ein Superoxid-Dismutase-Gen unter der Kontrolle eines Ascorbatoxidase-Promotors und ein Basta-resistentes Gen als einen Selektionsmarker umfasst,
b) Herstellung von Agrobacterium-Transformanten durch Transferieren von genanntem Vektor in Agrobacterium,
c) Cokultivierung genannter Agrobacterium-Transformanten mit Pflanzengewebe zur Verbesserung der Induktion von Adventivsprossen, worin genanntes Cokultivierungsverfahren Folgendes umfasst:
(i) Abtrennen des Hypokotyls von gekeimten Sämlingen zum Eliminieren des unteren Teils der Sämlinge,
(ii) Infizieren des Hypokotyl-Abschnitts der abgetrennten Sämlinge mit Agrobacterium-Transformanten,
(iii) Kultivieren der abgetrennten Sämlinge auf einem Medium zur Induktion von Adventivsprossen, wobei der Hypokotyl-Abschnitt das Medium kontaktiert, worin das Medium MS-Medium, ergänzt mit Zeatin, darstellt und
d) Regeneration des transformierten Gewebes zu adulten transgenen Pflanzen.

2. Verfahren zur Herstellung transgener Gurkenpflanzen nach Anspruch 1, worin genanntes Superoxid-Dismutase-Gen die Nukleinsäuresequenz von SEQ ID NO. 1 darstellt, die aus Cassava isoliert wird.

3. Verfahren zur Herstellung transgener Gurkenpflanzen nach Anspruch 1, worin genannter Expressionsvektor ASOp+mSOD1/pGPTV-Bar darstellt, welcher das Superoxid-Dismutase-Gen unter der Kontrolle eines Ascorbatoxidase-Promotors und das Basta-resistente Gen als einen Selektionsmarker umfasst.

4. Verfahren zur Herstellung transgener Gurkenpflanzen nach einem der vorangehenden Ansprüche, worin genannter *Agrobacterium*-Transformant *Agrobacterium tumefaciens* LBA 4404 darstellt, dem vom Koreanischen Forschungsinstitut für Biowissenschaft und Biotechnologie die Zugriffsnummer KCTC 0585BP zugewiesen wurde.

5. Verfahren zur Herstellung transgener Gurkenpflanzen nach einem der vorangehenden Ansprüche, worin genanntes Regenerationsverfahren die folgenden Schritte umfasst:
a) Induktion von Adventivsprossen aus dem infizierten Hypokotyl-Abschnitt auf Selektivmedium, worin das Selektivmedium MS-Medium, ergänzt mit 2,0 mg/l Zeatin, 300 mg/l Claforan und 2,0 mg/l Basta, darstellt,
b) Durchwurzelung der Adventivsprossen, die in a) auf einem Medium zur Wurzelinduktion induziert werden, worin das Medium zur Wurzelinduktion MS-Medium, ergänzt mit 1 mg/l IAA, darstellt und
c) Akklimatisierung der bewurzelten Pflänzchen an den Boden.

6. Verfahren zur Herstellung transgener Gurkenpflanzen nach einem der vorangehenden Ansprüche, worin genanntes oberes Hypokotyl abgetrennt wird, um eine Länge von 2 - 3 mm zurückzulassen.

7. Verfahren zur Herstellung transgener Gurkenpflanzen nach einem der vorangehenden Ansprüche, worin genannte gekeimte Sämlinge 3 - 5 Tage nach der Keimung abgetrennt werden und ein intaktes Kotyledon oder zwei halbexzidierte Kotyledonen aufweisen.

8. Transgene Gurkenpflanze, die mit einem Expressionsvektor transformiert ist, umfassend ein Superoxid-Dismutase-Gen unter der Kontrolle eines Ascorbatoxidase-Promotors und das Basta-resistente Gen als einen Selektionsmarker.

9. Verwendung der transgenen Pflanze nach Anspruch 8 als ein Material für Kosmetika, einschließlich Massage-Packungen oder als einen Zusatzstoff in funktionellen Nahrungsmitteln.

## Revendications

1. Méthode pour produire des plants de concombre transgéniques comprenant les étapes suivantes :
a) réalisation d'un vecteur d'expression, constitué du gène de la superoxyde dismutase sous le contrôle d'un promoteur de l'ascorbate oxydase et avec le gène de résistance au Basta comme marqueur de sélection ;
b) réalisation de transformants d'Agrobacterium par transfert dudit vecteur dans Agrobacterium ;
c) coculture desdits transformants d'Agrobacterium avec un tissu végétal afin d'améliorer l'induction des pousses adventices, ledit processus de coculture comprenant :
(i) la découpe de l'hypocotyle des plantules germés afin d'éliminer la partie inférieure des plantules ;
(ii) l'infection des segments hypocotyles des plantules découpés avec les transformants Agrobacterium ;
(iii) la mise en culture des plantules coupés sur un milieu pour l'induction de pousses adventices, le segment hypocotyle en contact avec le milieu, ce milieu étant un milieu MS additionné de zéatine ;
d) régénération du tissu transformé en plantes transgéniques adultes.

2. Méthode de production de plants de concombre transgéniques selon la revendication 1, ledit gène de la superoxyde transmutase étant la séquence d'acides nucléiques SEQ ID N°1, qui est isolée du manioc.

3. Méthode de production de plants de concombre transgéniques selon la revendication 1, ledit vecteur d'expression étant ASOp+mSOD1/pGPTV-Bar qui comprend le gène de la superoxyde dismutase sous le contrôle d'un promoteur de l'ascorbate oxydase et le gène de résistance au Basta en tant que marqueur de sélection.

4. Méthode de production de plants de concombre transgéniques selon n'importe quelle revendication précédente, ledit transformant d'Agrobacterium étant Agrobacterium tumefaciens LBA 4404 auquel a été assigné le n° d'accession KCTC 0585BP par le Korean Research Institute of Bioscience and Biotechnology.

5. Méthode de production de plants de concombre transgéniques selon n'importe quelle revendication précédente, ledit processus de régénération comprenant les étapes suivantes :
a) induction de pousses adventices à partir à partir du segment hypocotyle infecté sur un milieu sélectif, ledit milieu sélectif étant un milieu MS additionné de 2,0 mg/l de zéatine, de 300 mg/l de claforan et de 2,0 mg/l de Basta,
b) enracinement des pousses adventices induit dans a) sur un milieu d'induction des racines, ledit milieu d'induction des racines étant un milieu MS additionné de 1 mg/l d'IAA, et
c) acclimatation du plantule enraciné dans un sol.

6. Méthode de production de plants de concombre transgéniques selon n'importe quelle revendication précédente, ledit hypocotyle supérieur étant coupé de manière à laisser une longueur de 2 à 3 mm.

7. Méthode de production de plants de concombre transgéniques selon n'importe quelle revendication précédente, lesdits plantules germés étant détachés 3 à 5 jours après la germination, et ayant un cotylédon intact ou deux cotylédons semi-excisés.

8. Plant de concombre transgénique transformé avec un vecteur d'expression comprenant le gène de la superoxyde dismutase sous le contrôle d'un promoteur de l'ascorbate oxydase et avec le gène de résistance au Basta comme marqueur de sélection.

9. Utilisation de la plante tansgénique selon la revendication 8 en tant que matière première pour cosmétiques y compris produits de massage, ou en tant qu'additif dans des aliments fonctionnels.


### SEQUENCE LISTING

(1) GENERAL INFORMATION
   (iii) NUMBER OF SEQUENCE : 10
(2) INFORMATION FOR SEQ ID NO: 1:
   (i) SEQUENCE CHARACTERISTICS :
      (A) LENGTH : 801 base pairs
      (B) TYPE : nucleic acid
   (ii) MOLECULAR TYPE : cDNA
   (iii) ORIGINAL SOURCE :
      (A) ORGANISM : Manihot esculenta
   (iv) FEATURE :
      (A) NAME/KEY : CDS
      (B) LOCATION : (55)..(510)
   (iv) FEATURE :
      (A) NAME/KEY : polyA
      (B) LOCATION : (781)..(801)
   (iv) FEATURE :
      (A) NAME/KEY : polyA_signal
      (B) LOCATION : (611)..(616)
   (v) SEQUENCE DESCRIPTION : SEQ ID NO: 1:
   tctcgatctt ctctgtctaa gctctaaagg ggtgctctga gatcacgtaa aaca          54
   atg gtg aag gct gaa gct gtt ctt acc agt agt gag ggg gtt agc gga      102
   Met Val Lys Ala Glu Ala Val Leu Thr Ser Ser Glu Gly Val Ser Gly
    1               5                  10                  15
   aca atc ttc ttt acc caa gaa gga gat ggt cct acc act gta act gga      150
   Thr Ile Phe Phe Thr Gln Glu Gly Asp Gly Pro Thr Thr Val Thr Gly
                 20                  25                  30
   aac att tcc ggc ctt aag cca ggg ctt cat ggg ttc cac gtc cat gcc      198
   Asn Ile Ser Gly Leu Lys Pro Gly Leu His Gly Phe His Val His Ala
           35                  40                  45
   ctt gga gac aca aca aac ggt tgc atg tca act ggg cca cac ttt aac      246
   Leu Gly Asp Thr Thr Asn Gly Cys Met Ser Thr Gly Pro His Phe Asn
       50                  55                  60
   cct tct ggc aaa gat cat ggt gcc cct gag gat gag att cgt cat gct      294
   Pro Ser Gly Lys Asp His Gly Ala Pro Glu Asp Glu Ile Arg His Ala
   65                  70                  75                  80
   ggt gat ctg gga aat gtc act gct ggt gat gat ggc act gct agt ttc      342
   Gly Asp Leu Gly Asn Val Thr Ala Gly Asp Asp Gly Thr Ala Ser Phe
                   85                  90                  95
   aca att att gac aag cat att cct ctt tct ggt caa aat tca atc ata      390
   Thr Ile Ile Asp Lys His Ile Pro Leu Ser Gly Gln Asn Ser Ile Ile
               100                 105                 110
   gga agg gca gtt gtt gtt cat gca gat cct gat gat ctt ggc agg gga      438
   Gly Arg Ala Val Val Val His Ala Asp Pro Asp Asp Leu Gly Arg Gly
           115                 120                 125
   gga cat gaa ctc agt aaa acc acc gga aat gct ggt ggc aga gta gca      486
   Gly His Glu Leu Ser Lys Thr Thr Gly Asn Ala Gly Gly Arg Val Ala
       130                 135                 140
   tgc ggt att att ggt ttg cga gga  tagagtgctt ctccagagat caataacaag    540
   Cys Gly Ile Ile Gly Leu Arg Gly
   145                 150
   acaaagacag ctgaaacatg cacagccgga caacctttag aagaacgtta ggagaccatt    600
   aactcatttg aataaaagaa agaataatac tgtagttttg gctggtttgg tcttgtgatc     660
   tcaagatggt gtatgctttg tatggtttcg tgaagtttat tgaactttga actttttcga     720
   atggtagggc ttgctctttg tctggtccaa attcaggccg tggatgtttt atactgcttt     780
   aaaaaaaaaa aaaaaaaaaa a                                               801
(3) INFORMATION FOR SEQ ID NO: 2:
   (i) SEQUENCE CHARACTERISTICS :
      (A) LENGTH : 152 amino acids
      (B) TYPE : amino acid
   (ii) MOLECULAR TYPE : protein
   (iii) ORIGINAL SOURCE :
      (A) ORGANISM : Manihot esculenta
   (iv) SEQUENCE DESCRIPTION : SEQ ID NO: 2:
   Met Val Lys Ala Glu Ala Val Leu Thr Ser Ser Glu Gly Val Ser Gly
    1              5                  10                  15
   Thr Ile Phe Phe Thr Gln Glu Gly Asp Gly Pro Thr Thr Val Thr Gly
               20                  25                  30
   Asn Ile Ser Gly Leu Lys Pro Gly Leu His Gly Phe His Val His Ala
           35                  40                  45
   Leu Gly Asp Thr Thr Asn Gly Cys Met Ser Thr Gly Pro His Phe Asn
       50                  55                  60
   Pro Ser Gly Lys Asp His Gly Ala Pro Glu Asp Glu Ile Arg His Ala
   65                  70                  75                  80
   Gly Asp Leu Gly Asn Val Thr Ala Gly Asp Asp Gly Thr Ala Ser Phe
                   85                  90                  95
   Thr Ile Ile Asp Lys His Ile Pro Leu Ser Gly Gln Asn Ser Ile Ile
               100                 105                 110
   Gly Arg Ala Val Val Val His Ala Asp Pro Asp Asp Leu Gly Arg Gly
                   115                 120                 125
   Gly His Glu Leu Ser Lys Thr Thr Gly Asn Ala Gly Gly Arg Val Ala
       130                 135                 140
   Cys Gly Ile Ile Gly Leu Arg Gly
   145                 150
(4) INFORMATION FOR SEQ ID NO: 3:
   (i) SEQUENCE CHARACTERISTICS :
      (A) LENGTH : 20 base pairs
      (B) TYPE : nucleic acid
   (ii) STRANDEDNESS : single
   (iii) TOPOLOGY : linear
   (iv) MOLECULAR TYPE : other nucleic acid (synthetic oligonucleotide)
   (v) FEATURE :
      (A) OTHER INFORMATION : degenerate primer SODF1
   (vi) SEQUENCE DESCRIPTION : SEQ ID NO: 3:
   cttggncttc atggnttcca                                              20
(5) INFORMATION FOR SEQ ID NO: 4:
   (i) SEQUENCE CHARACTERISTICS :
      (A) LENGTH : 20 base pairs
      (B) TYPE : nucleic acid
   (ii) STRANDEDNESS : single
   (iii) TOPOLOGY : linear
   (iv) MOLECULAR TYPE : other nucleic acid (synthetic oligonucleotide
   (v) FEATURE :
      (A) OTHER INFORMATION : degenerate primer SODR2
   (vi) SEQUENCE DESCRIPTION : SEQ ID NO: 4:
   ctnccaccag catttccagt                                              20
(6) INFORMATION FOR SEQ ID NO: 5:
   (i) SEQUENCE CHARACTERISTICS :
      (A) LENGTH : 32 base pairs
      (C) TYPE : nucleic acid
   (ii) STRANDEDNESS : single
   (iii) TOPOLOGY : linear
   (iv) MOLECULAR TYPE : other nucleic acid (synthetic oligonucleotide)
   (v) FEATURE :
      (A) OTHER INFORMATION : HindIII-tag PCR primer for ASOp
   (vi) SEQUENCE DESCRIPTICN : SEQ ID NO: 5:
   gcgcaagctt ctaaatattc tcttttaatt tg                                32
(7) INFORMATION FOR SEQ ID NO: 6:
   (i) SEQUENCE CHARACTERISTICS :
      (A) LENGTH : 24 base pairs
      (B) TYPE : nucleic acid
   (ii) STRANDEDNESS : single
   (iii) TOPOLOGY : linear
   (iv) MOLECULAR TYPE : other nucleic acid (synthetic oligonucleotide)
   (v) FEATURE :
      (A) OTHER INFORMATION : EcoRI-tag PCR primer for ASOp
   (vi) SEQUENCE DESCRIPTION : SEQ ID NO: 6
   gcgcgaattc ttcgaagggt tgag                                         24
(8) INFORMATION FOR SEQ ID NO: 7:
   (i) SEQUENCE CHARACTERISTICS :
      (A) LENGTH : 25 base pairs
      (B) TYPE : nucleic acid
   (ii) STRANDEDNESS : single
   (iii) TOPOLOGY : linear
   (iv) MOLECULAR TYPE : other nucleic acid (synthetic oligonucleotide)
   (v) FEATURE : PstI-tag PCR primer for mSOD1
      (A) OTHER INFORMATION :
   (vi) SEQUENCE DESCRIPTION : SEQ ID NO: 7:
   gcgcctgcag tctcgatctt ctctg                                        25
(9) INFORMATION FOR SEQ ID NO: 8:
   (i) SEQUENCE CHARACTERISTICS :
      (A) LENGTH : 25 base pairs
      (B) TYPE : nucleic acid
   (ii) STRANDEDNESS : single
   (iii) TOPOLOGY : linear
   (iv) MOLECULAR TYPE : other nucleic acid (synthetic oligonucleotide)
   (v) FEATURE :
      (A) OTHER INFORMATION : BamHI-tag PCR primer for mSOD1
   (vi) SEQUENCE DESCRIPTION : SEQ ID NO: 8:
   gcgcggatcc aacatccacg gcctg                                    25
(10) INFORMATION FOR SEQ ID NO: 9:
   (i) SEQUENCE CHARACTERISTICS :
      (A) LENGTH : 20 base pairs
      (B) TYPE : nucleic acid
   (ii) STRANDEDNESS : single
   (iii) TOPOLOGY : linear
   (iv) MOLECULAR TYPE : other nucleic acid (synthetic oligonucleotide)
   (v) FEATURE :
      (A) OTHER INFORMATION : Bar primer 1
   (vi) SEQUENCE DESCRIPTION : SEQ ID NO: 9:
   ggtctgcacc atcgtcaacc                                          20
(11) INFORMATION FOR SEQ ID NO: 10:
   (1) SEQUENCE CHARACTERISTICS :
      (A) LENGTH : 20 base pairs
      (B) TYPE : nucleic acid
   (ii) STRANDEDNESS : single
   (iii) TOPOLOGY : linear
   (iv) MOLECULAR TYPE : other nucleic acid (synthetic oligonucleotide)
   (v) FEATURE :
      (A) OTHER INFORMATION : Bar primer 2
   (vi) SEQUENCE DESCRIPTION : SEQ ID NO: 10:
   tcagatctcg gtgacgggca                                          20